# EUROPEAN PATENT SPECIFICATION

(11) **EP 0 836 472 B1**
(45) Date of publication and mention of the grant of the patent: **22.08.2001**
(21) Application number: 96922906.1
(22) Date of filing: 28.06.1996
(51) Int. Cl.: A61K 9/00

(54) **THERAPEUTIC EFFERVESCENT COMPOSITIONS**
THERAPEUTISCHE BRAUSEMISCHUNG
COMPOSITIONS EFFERVESCENTES THERAPEUTIQUES

(30) Priority: 05.07.1995 FR 9508116
(43) Date of publication of application: 22.04.1998
(73) Proprietor: SMITHKLINE BEECHAM LABORATOIRES PHARMACEUTIQUES, 92731 Nanterre Cédex (FR)
(72) Inventor: MENTION, Jacky, Andre, Gustave, F-53101 Mayenne Cédex (FR)
(74) Representative: Connell, Anthony Christopher
(86) International application number: EP9602934
(87) International publication number: WO9702014

(56) References cited:
- EP-A- 0 186 090
- EP-A- 0 233 853
- EP-A- 0 265 951
- EP-A- 0 414 115

## Description

This invention relates to novel granulates comprising effervescent couples, pharmaceutical formulations comprising such granulates, to processes for the manufacture thereof and the use of such formulations in therapy.

Effervescent pharmaceutical formulations are well known in the art. At the simplest level, such compositions include a couple which comprises an acid such as citric acid or a mono or dihydrogen salt thereof and a carbon dioxide source such as a carbonate or bicarbonate alkali metal salt, such as sodium bicarbonate. These do not react together when dry but combine to release carbon dioxide and an effervescent effect in the presence of water. The pharmaceutical compositions may be in the form of a tablet for dissolving in water or a dispersible powder for sprinkling onto water, prior to administration. The components of the couple are blended together during manufacture of the composition. It is important to avoid water, to prevent premature effervescence. The latter, in the case of a citric acid or mono hydrogen salt/sodium bicarbonate couple, leads to the formation of carbon dioxide and may lead to additional and undesirable degradation when the drug substance is acid sensitive.

A suitable drug for use in an effervescent formulation is cimetidine, marketed by Smith Kline & French Laboratories as Tagamet. An effervescent tablet preparation containing 200mg cimetidine and a citric acid/sodium bicarbonate stoichiometric couple is marketed in several European countries. The couple is produced by a wet granulation process. The effervescent formulation also helps to overcome the bitter taste of cimetidine. In addition, EP 0 233 853-A (Laboratoires Smith Kline & French) describes effervescent couples particularly suitable for use with H₂ antagonists such as cimetidine which are sensitive to acid. The couple comprises a mixture of mono- and dialkali metal citrate salts in a defined ratio. This is prepared by partially neutralising citric acid by treatment thereof with an alkali metal carbonate or bicarbonate salt in water and stopping the reaction when a certain amount of carbon dioxide has been evolved. This processes involves several steps, including wet granulation and subsequent drying. A shorter, more efficient process would be commercially attractive.

We have now found that improved couples may be prepared more efficiently if a dry granulation technique involving a roller compactor is used to blend together the components of the couple. The use of a roller compactor is becoming increasingly common in pharmaceutical technology. The technique depends upon the densification of a fine powder to obtain fragments or flakes of undefined shape.
These flakes are then usually crushed to obtain granulates which are screened to the size required by the user.

The use of roller compaction to prepare granulates comprising a sodium hydrogen sulphate/sodium bicarbonate couple, for subsequent incorporation into effervescent tablets for use in cleaning toilet bowls, is described in Pharmaceutical Dosage Forms : Tablets, vol. 1, ed. H.A. Lieberman and L. Lachman (Marcel Dekkel, 1980).

Accordingly, the present invention provides for pharmaceutical granulates comprising an effervescent couple, characterised in that the couple comprises anhydrous powdered monosodium citrate and powdered sodium bicarbonate and the granulates are prepared using a roller compactor.

Such granulates are found to dissolve more quickly and more fully in water and there is also no chemical reaction between the components.

The most effective granulates are made using anhydrous powdered monosodium citrate of a certain particle size range. Suitable grades of anhydrous powdered monosodium citrate comprise particles which are substantially (i.e. about more than 90%) within the range 0 to 500 microns, preferably 355 microns, more preferably 0 to 250 microns. Suitable grades are available from Roche (monosodium citrate anhydrous powder, maximum of 5%w/w with grain size >0.250mm), Boehringer Ingelheim (monosodium zitrate wasserfrei Art-Nr 661 511, minimum of 90%w/w with grain size < 0.150mm), Jungbunzlauer (maximum of 5%w/w with grain size >0.355mm) and Haarmann & Reimer Corp. (maximum of 1% with grain size >0.500mm).

Suitable grades of powdered sodium bicarbonate comprise particles which are substantially (i.e. about more than 90%) within the range of 0 to 500 microns, preferably 270 microns, more preferably 0 to 130 microns. Suitable grades of powdered sodium bicarbonate are available from Solvay, for instance the grades 0 to 13 (particle size, by sieving method: >0.16mm max; 15g/kg) and extra-fine (particle size by sieving method: >0.125mm max; 20g/kg).

Tablets manufactured using granulates with low particle size were found to be harder than those manufactured using granulates with large particle size but were otherwise comparable.

Granulates according to the present invention may be obtained by a process which comprises the steps of:
a) blending together the components of the couple;
b) roller compacting the blend to produce flakes;
c) crushing the flakes to obtain granulates; and
d) if desired, screening the granulates by size.

Suitably, the components of the couple are blended together at a controlled temperature, for instance about 20°C and controlled relative humidity, for instance about 20% RH and processed in a roller compactor to form flakes. Roller compaction may be carried out over a range of linear compaction strengths, the appropriate value being influenced by the particle size of the monosodium citrate and sodium bicarbonate. For smaller particle sizes (for instance, the grades of monosodium citrate from Boehringer Ingelheim and sodium bicarbonate from Solvay previously mentioned), linear roller compaction strengths of about 20 KN/cm are found to give suitable granulates for further processing into final finished forms such as tablets. For larger particle sizes (for instance monosodium citrate from Jungbunzlauer), linear compaction strengths of about 30 KN/cm are preferred. The flakes thus obtained are then crushed to obtain granulates which are screened to the size required for further processing, suitably a size in the range 0 to 1250 microns is used. Suitably the granulates thus obtained are calibrated for effervescent potency using a standard assay, prior to further use.

Suitably, granulates according to the present invention are incorporated into a pharmaceutical formulation. Accordingly, in a further aspect, the present invention provides a pharmaceutical formulation comprising granulates comprising an effervescent couple and a drug substance which may optionally be incorporated into the granulates, characterised in that the couple comprises anhydrous powdered monosodium citrate and powdered sodium bicarbonate and the granulates are processed using a roller compactor.

Suitably, granulates may comprise the components of the couple and, optionally, other pharmaceutically acceptable excipients and/or drug substance. Accordingly, in a further aspect, the present invention provides pharmaceutical formulations comprising granulates comprising an effervescent couple and a drug substance, characterised in that the couple comprises anhydrous powdered monosodium citrate and powdered sodium bicarbonate and the granulates are prepared using a roller compactor.

Suitable drug substances include H₂-antagonists well known in the art such as ranitidine, cimetidine, famotidine, nizatidine and roxatine or pharmaceutically acceptable salts thereof. A representative drug substance is cimetidine or the hydrochloride salt thereof. Other suitable drug substances include those obtainable directly 'over the counter' (OTC drugs) such as analgesics, for instance aspirin, ketoprofen, naproxen, paracetamol and ibuprofen. Other suitable products in which such effervescent couples may be incorporated include indigestion products, vitamin supplements and antibiotics.

Suitably, the drug substance is incorporated in an amount such that individual tablets comprise unit dosages of the particular medicament, for instance 200mg of cimetidine or 500mg of paracetamol.

Suitable pharmaceutical formulations include effervescent tablets and sachets containing water dispersible powders.

Pharmaceutical formulations according to the present invention may be prepared by blending together the granulates formed by roller compaction with other components prior to processing into final form. Roller compaction may also be extended to include other components, such as drug substance and excipients such as lubricants, disintegrants, flavours and sweeteners. For tablets, final processing may include compressing into tablets using a tabletting machine.

Preferably, monosodium citrate and sodium bicarbonate, as herein before defined, are blended together and then roller compacted, preferably in the absence of water, to form flakes which are then crushed to give granulates. The granulates thus obtained may then be combined with drug substance, for instance cimetidine (or the hydrochloride salt thereof), conventional tabletting or filling agents and, optionally, sweeteners, flavours and lubricants and compressed or filled together, suitably under controlled ambient conditions, to form tablets or sachets, respectively. Suitable tablets will have a hardness in the range 6 to 12 Kp. The hardness of the final tablets is found to be influenced by the linear roller compaction strength used in preparing the granulates. Suitable linear compaction strengths are in turn influenced by the particle size of the monosodium citrate and sodium bicarbonate. For smaller particle sizes (for instance the grades of monosodium citrate from Boehringer Ingelheim and sodium bicarbonate from Solvay previously mentioned) a linear roller compaction strength of about 20KN/cm is preferred. Higher linear compaction strengths are found to granulates which when incorporated into tablets give harder tablets (up to 17Kp). At a linear compaction strength of less than 16KN/cm, subsequent tablets are found to be very soft. For larger particle sizes (for instance monosodium citrate from Jungbunzlauer), a linear compaction strength of about 30 KN/cm is preferred. Tablets comprising cimetidine show no cimetidine degradation problems after storage for 2 months at 40°C.

In addition, drug substance, for instance cimetidine, may also be blended together with the components of the couple and the mixture then subjected to roller compaction, followed by crushing to give granulates. These granulates may then be blended together with sweeteners, flavours and lubricants and then compressed into tablets. The sweeteners and flavours may also be incorporated into the initial blend, for roller compaction.

Suitable sweeteners are well known in the art and include aspartame, sodium saccharin, acesulfame potassium and sodium cyclamate.

Suitable lubricants are well known in the art and include PEG 6000, sodium benzoate and dimethicone.

The formulations of the present invention may also include additional excipients and agents well known in the art, for instance disintegrants, wetting agents and colouring agents.

Pharmaceutical formulations according to the present invention are of use in therapy according to the identity of the drug substance contained therein. Accordingly, in a further aspect, the present invention provides for a pharmaceutical formulation herein before defined for use in therapy. The present invention also provides for the use of a couple as herein before defined in the manufacture of a medicament for use in therapy. Such pharmaceutical formulations containing an H₂-antagonist such as cimetidine are of use in the treatment of duodenal, gastric, recurrent and stomach ulcerating reflux oesophagitis, and in the management of patients who are at high risk from haemorrhage of the upper gastro-intestinal tract.

The invention will now be illustrated by the following example.

### Example 1 - '200mg' effervescent tablet comprising cimetidine

### 1. Stoichiometric roller compacted effervescent granules

| | |
|---|---|
| Anhydrous powdered monosodium citrate (ex B Ingelheim) | 1426.2g |
| Powdered sodium bicarbonate (ex Solvay) | 1118.8 |

The ingredients were blended together and subjected to roller compaction at a linear compaction strength of 20 KN/cm at 20-25°C and about 20%RH. The flakes were then crushed and size-sorted by sieving to give granulates for use in tablet formulation.

### 2. Tablet

| | w/w |
|---|---|
| Stoichiometric roller compacted effervescent granulates | 2,545g |
| Cimetidine base | 200.0 |
| Flavours | 27.5 |
| Aspartame | 15.0 |
| Saccharin Sodium | 8.0 |
| PEG 6000 | 20.0 |
| Sodium benzoate | 100.0 |

The above ingredients, suitably scaled up, were blended together in a mixing device at 20-25°C and about 20%RH and the resultant blend tabletted on a rotative press fitted with 22mm punches. This gave flat round tablets weighing 2915.5mg with a hardness about 6 to 12Kp which dissolved in 45 to 75s in water at 20°C.

### Example 2 - '500mg' effervescent tablet comprising paracetamol

### 1. Stoichiometric roller compacted effervescent granules

| | |
|---|---|
| Anhydrous powdered monosodium citrate (ex Jungbunzlauer) | 1426.2g |
| Powdered sodium bicarbonate 0/13 (ex Solvay) | 1118.8 |

The ingredient were blended together and subjected to roller compaction at a linear compaction strength of 30 KN/cm at 20-25°C and about 20% RH. The flakes were then crushed and size-sorted by sieving through a 1.25mm screen to give granulates for use in tablet formulation.

### 2. Tablet

| | (w/w) |
|---|---|
| Stoichiometric roller compacted effervescent granules | 1840.0 mg |
| Paracetamol (powdered) | 500.0 mg |
| Sorbitol | 400.0 mg |
| PEG 6000 | 100.0 mg |
| Polysorbate 80 | 3.0 mg |
| Sodium saccharinate | 8.0 mg |
| (TOTAL | 2851.0 mg) |

The polysorbate was mixed with a part of sorbitol to give an homogeneous dry mixing.
After the above mixing, the remaining sorbitol and other components were blended together in a mixing device at 20-25°C and about 20% RH. The resultant blend was tabletted on a press fitted with 20mm punches.
The tablets obtained showed following characteristics:

| | |
|---|---|
| weight | 2851 mg |
| thickness | about 4.48mm |
| dissolution time | about 45 s |

## Claims

1. Pharmaceutical granulates comprising an effervescent couple, characterised in that the couple comprises anhydrous powdered monosodium citrate and powdered sodium bicarbonate and the granulates are prepared using a roller compactor.

2. A pharmaceutical formulation comprising granulates comprising an effervescent couple and a drug substance which may optionally be incorporated into the granulates, characterised in that the couple comprises anhydrous powdered monosodium citrate and powdered sodium bicarbonate and the granulates are prepared using a roller compactor.

3. Pharmaceutical granulates or formulations comprising such granulates as claimed in claim 1 or 2 in which the anhydrous powdered monosodium citrate has particles which are substantially within the size range 0 to 500 microns.

4. Pharmaceutical granulates or formulations comprising such granulates as claimed in claim 3 in which the anhydrous powdered monosodium citrate has particles which are substantially within the size range 0 to 355 microns.

5. Pharmaceutical granulates or formulations comprising such granulates as claimed in claim 4 in which the anhydrous powdered monosodium citrate has particles which are substantially within the size range 0 to 250 microns.

6. Pharmaceutical granulates or formulations comprising such granulates as claimed in any one of claims 1 to 5 in which the powdered sodium bicarbonate has particles which are substantially within the size range 0 to 500 microns.

7. Pharmaceutical granulates or formulations comprising such granulates as claimed in claim 6 in which the powdered sodium bicarbonate has particles which are substantially within the size range 0 to 270 microns.

8. Pharmaceutical granulates or formulations comprising such granulates as claimed in claim 7 in which the powdered sodium bicarbonate has particles which are substantially within the size range 0 to 130 microns.

9. Pharmaceutical granulates or a formulation as claimed in any one of claims 1 to 8 in which the drug substance is an H₂-antagonist or an analgesic.

10. A pharmaceutical granulate or a formulation comprising such granulates as claimed in claim 9 in which the drug substance is cimetidine or the hydrochloride salt thereof or paracetamol.

11. A pharmaceutical formulation as claimed in any one of claims 2 to 10 in the form of a tablet or a water dispersible powder.

12. A process for preparing pharmaceutical granulates as defined in any one of claims 1 and 3 to 9 which process comprises the steps of:
a) blending together the components of the couple; and
b) roller compacting the blend to produce flakes;
c) crushing the flakes to obtain granulates; and
d) if necessary and so desired, screening the granulates by size.

13. A process for preparing a pharmaceutical formulation as defined in any one of claims 2 to 11 which comprises admixing granulates as defined in any one of claims 1 to 10 with excipients and optionally drug substance in appropriate quantities and in any order.

14. The use of granulates comprising an effervescent couple characterised in that the couple comprises anhydrous powdered monosodium citrate and powdered sodium bicarbonate prepared by roller compaction in the manufacture of a medicament for use in therapy.

## Patentansprüche

1. Pharmazeutische Granulate, umfassend ein schäumendes Paar, dadurch gekennzeichnet, daß das Paar wasserfreies pulverförmiges Mononatriumcitrat und pulverförmiges Natriumbicarbonat umfaßt und die Granulate unter Verwendung eines Walzenverdichters hergestellt werden.

2. Pharmazeutische Formulierung, umfassend Granulate, die ein schäumendes Paar und eine Arzneimittelsubstanz umfassen, welche gegebenenfalls in die Granulate eingeschlossen sein kann, dadurch gekennzeichnet, daß das Paar wasserfreies pulverförmiges Mononatriumcitrat und pulverförmiges Natriumbicarbonat umfaßt und die Granulate unter Verwendung eines Walzenverdichters hergestellt werden.

3. Pharmazeutische Granulate oder solche Granulate umfassende Formulierungen nach Anspruch 1 oder 2, wobei das wasserfreie pulverförmige Mononatriumcitrat Teilchen aufweist, die im wesentlichen innerhalb des Größenbereichs von 0 bis 500 Mikron liegen.

4. Pharmazeutische Granulate oder solche Granulate umfassende Formulierungen nach Anspruch 3, wobei das wasserfreie pulverförmige Mononatriumcitrat Teilchen aufweist, die im wesentlichen innerhalb des Größenbereichs von 0 bis 355 Mikron liegen.

5. Pharmazeutische Granulate oder solche Granulate umfassende Formulierungen nach Anspruch 4, wobei das wasserfreie pulverförmige Mononatriumcitrat Teilchen aufweist, die im wesentlichen innerhalb des Größenbereichs von 0 bis 250 Mikron liegen.

6. Pharmazeutische Granulate oder solche Granulate umfassende Formulierungen nach einem der Ansprüche 1 bis 5, wobei das pulverförmige Natriumbicarbonat Teilchen aufweist, die im wesentlichen innerhalb des Größenbereichs von 0 bis 500 Mikron liegen.

7. Pharmazeutische Granulate oder solche Granulate umfassende Formulierungen nach Anspruch 6, wobei das pulverförmige Natriumbicarbonat Teilchen aufweist, die im wesentlichen innerhalb des Größenbereichs von 0 bis 270 Mikron liegen.

8. Pharmazeutische Granulate oder solche Granulate umfassende Formulierungen nach Anspruch 7, wobei das pulverförmige.Natriumbicarbonat Teilchen aufweist, die im wesentlichen innerhalb des Größenbereichs von 0 bis 130 Mikron liegen.

9. Pharmazeutische Granulate oder Formulierung nach einem der Ansprüche 1 bis 8, wobei die Arzneimittelsubstanz ein H₂-Antagonist oder ein Analgetikum ist.

10. Pharmazeutisches Granulat oder solche Granulate umfassende Formulierung nach Anspruch 9, wobei die Alzneimittelsubstanz Cimetidin oder das Hydrochloridsalz davon oder Paracetamol ist.

11. Pharmazeutische Formulierung nach einem der Ansprüche 2 bis 10 in Form einer Tablette oder eines wasserdispergierbaren Pulvers.

12. Verfahren zur Herstellung pharmazeutischer Granulate nach einem der Ansprüche 1 und 3 bis 9, das die folgenden Schritte umfaßt:
a) Zusammenmischen der Komponenten des Paars; und
b) Walzenverdichten der Mischung, um Flocken herzustellen;
c) Zerstoßen der Flocken, um Granulate zu erhalten; und
d) falls erforderlich und gewünscht, Sieben der Granulate nach Größe.

13. Verfahren zur Herstellung eines pharmazeutischen Formulierung nach einem der Anspräche 2 bis 11, welches das Mischen von Granulaten nach einem der Ansprüche 1 bis 10 mit Arzneimittelträgern und gegebenenfalls Arzneimittelsubstanz in geeigneten Mengen und in beliebiger Reihenfolge umfaßt.

14. Verwendung von Granulaten, umfassend ein schäumendes Paar, dadurch gekennzeichnet, daß das Paar durch Walzenverdichten hergestelltes wasserfreies pulverförmiges Mononatriumcitrat und pulveriförmiges Natriumbicarbonat umfaßt, bei der Herstellung eines Medikaments zur Verwendung bei der Therapie.

## Revendications

1. Granulés pharmaceutiques comprenant un couple effervescent, caractérisés en ce que le couple comprend du citrate monosodique pulvérisé anhydre et du bicarbonate de sodium pulvérisé et les granulés sont préparés au moyen d'un compacteur à rouleaux.

2. Formulation pharmaceutique comprenant des granulés comprenant un couple effervescent et une substance médicamenteuse qui peur être facultativement incorporée aux granulés, caractérisée en ce que le couple comprend du citrate monosodique pulvérisé anhydre et du bicarbonate de sodium pulvérisé et les granulés sont préparés au moyen d'un compacteur à rouleaux.

3. Granulés pharmaceutiques ou formulations comprenant de tels granulés suivant la revendication 1 ou 2, dans lesquels le citrate monosodique pulvérisé anhydre a des particules qui sont comprises essentiellement dans l'intervalle de dimensions de 0 à 500 micromètres.

4. Granulés pharmaceutiques ou formulations comprenant de tels granulés suivant la revendication 3, dans lesquels le citrate monosodique pulvérisé anhydre a des particules qui sont comprises essentiellement dans l'intervalle de dimensions de 0 à 355 micromètres.

5. Granulés pharmaceutiques ou formulations comprenant de tels granulés suivant la revendication 4, dans lesquels le citrate monosodique pulvérisé anhydre a des particules qui sont comprises essentiellement dans l'intervalle de dimensions de 0 à 250 micromètres.

6. Granulés pharmaceutiques ou formulations comprenant de tels granulés suivant l'une quelconque des revendications 1 à 5, dans lesquels le bicarbonate de sodium pulvérisé a des particules qui sont comprises essentiellement dans l'intervalle de dimensicns de 0 à 500 micromètres.

7. Granulés pharmaceutiques ou formulations comprenant de tels granulés suivant la revendication 6, dans lesquels le bicarbonate de sodium pulvérisé a des particules qui sont comprises essentiellement dans l'intervalle de dimensions de 0 à 270 micromètres.

8. Granulés pharmaceutiques ou formulations comprenant de tels granulés suivant la revendication 7, dans lesquels le bicarbonate de sodium pulvérisé a des particules qui sont comprises essentiellement dans l'intervalle de dimensions de 0 à 130 micromètres.

9. Granulés pharmaceutiques ou formulation suivant l'une quelconque des revendications 1 à 8, dans lesquels la substance médicamenteuse consiste en un antagoniste H₂ ou un analgésique.

10. Granulés pharmaceutiques ou formulation comprenant de tels granulés suivant la revendication 9, dans lesquels la substance médicamenteuse est la cimétidine ou son chlorhydrate ou bien le paracétamol.

11. Formulation pharmaceutique suivant l'une quelconque des revendications 2 à 10 sous forme d'un comprimé ou d'une poudre dispersable dans l'eau.

12. Procédé pour la préparation de granulés pharmaceutiques répondant à la définition suivant l'une quelconque des revendications 1 et 3 à 9, procédé qui comprend les étapes consistant :
a) à mélanger ensemble les constituants du couple ; et
b) à compacter au rouleau le mélange pour produire des paillettes ;
c) à broyer les paillettes pour obtenir des granulés ; et
d) si nécessaire et si cela est désiré, à tamiser les granulés en fonction des dimensions.

13. Procédé pour la préparation d'une formulation pharmaceutique répondant à la définition suivant l'une quelconque des revendications 2 à 11, qui comprend le mélange de granulés répondant à la définition suivant l'une quelconque des revendications 1 à 10 à des excipients et, facultativement, une substance médicamenteuse en des quantités appropriées et dans n'importe quel ordre.

14. Utilisation de granulés comprenant un couple effervescent, caractérisée en ce que le couple comprend du citrate monosodique pulvérisé anhydre et du bicarbonate de sodium pulvérisé préparé par compactage au rouleau dans la production d'un médicament destiné à être utilisé en thérapeutique.
